(19) Europäisches Patentamt
European Patent Office
Office européen des brevets

(11) Veröffentlichungsnummer: **0 161 480 B1**

(12) # EUROPÄISCHE PATENTSCHRIFT

(45) Veröffentlichungstag der Patentschrift: **03.06.87**

(51) Int. Cl.⁴: **C 07 C 143/75**, A 01 N 41/06

(21) Anmeldenummer: **85104259.8**

(22) Anmeldetag: **09.04.85**

(54) **N-Jodpropargyl-chlormethansulfonamide.**

(30) Priorität: **18.04.84 DE 3414600**

(43) Veröffentlichungstag der Anmeldung: **21.11.85 Patentblatt 85/47**

(45) Bekanntmachung des Hinweises auf die Patenterteilung: **03.06.87 Patentblatt 87/23**

(84) Benannte Vertragsstaaten: **AT BE CH DE FR GB IT LI NL SE**

(56) Entgegenhaltungen: **GB - A - 2 041 371**

(73) Patentinhaber: **BAYER AG, Konzernverwaltung RP Patentabteilung, D-5090 Leverkusen 1 Bayerwerk (DE)**

(72) Erfinder: **Oeckl, Siegfried, Dr., Auf der Höhe 74, D-5060 Bergisch-Gladbach (DE)**
Erfinder: **Schade, Gerold, Dr., Hahnenweg 8, D-5000 Köln 80 (DE)**
Erfinder: **Brandes, Wilhelm, Dr., Eichendorffstrasse 3, D-5653 Leichlingen 1 (DE)**
Erfinder: **Reinecke, Paul, Dr., Lessingstrasse 11, D-5090 Leverkusen 3 (DE)**
Erfinder: **Paulus, Wilfried, Dr., Deswatinesstrasse 90, D-4150 Krefeld 1 (DE)**

**EP 0 161 480 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue N-Jodpropargyl-chlormethansulfonamide, Verfahren zu ihrer Herstellung und ihre Verwendung als Schädlingsbekämpfungsmittel.

Es ist bereits bekannt, dass eine Reihe von organischen Verbindungen, wie z.B. Zinkethylen-1,2-bis-dithiocarbamat, fungizide Eigenschaften aufweisen (vgl. z.B. R. Wegler, „Chemie der Pflanzenschutz- und Schädlingsbekämpfungsmittel", Springer-Verlag, Berlin-Heidelberg-New York, 1970, Bd. 2, S. 20).

Ferner sind N-Jodpropargyl-sulfonamide, wie z.B. N-Jodpropargyl-(4-methylphenyl)-sulfonamid, bekannt. Diese Verbindungen besitzen antimikrobielle Eigenschaften (vgl. Jap. Pat. 49100-037).

Es wurden neue N-Jodpropargyl-chlormethansulfonamide der allgemeinen Formel (I)

$Cl-CH_2-SO_2-N-CH_2-C\equiv CJ$ (I)
|
R

in welcher

R für Alkyl oder für gegebenenfalls ein- bis mehrfach, gleich oder verschieden durch Alkyl, Halogen und Halogenalkyl substituiertes Aryl steht, gefunden.

Weiterhin wurde gefunden, dass man die neuen N-Jodpropargyl-chlormethansulfonamide der Formel (I)

$Cl-CH_2-SO_2-N-CH_2-C\equiv CJ$ (I)
|
R

in welcher

R für Alkyl oder für gegebenenfalls ein- bis mehrfach, gleich oder verschieden durch Alkyl, Halogen und Halogenalkyl substituiertes Aryl steht, erhält, wenn man N-Propargyl-chlormethansulfonamide der Formel (II)

$Cl-CH_2-SO_2-N-CH_2-C\equiv CH$ (II)
|
R

in welcher

R die oben angegebene Bedeutung hat, in Gegenwart einer Base und gegebenenfalls in Gegenwart eines Lösungs- oder Verdünnungsmittels mit Jod umsetzt.

Die neuen N-Jodpropargyl-chlormethansulfonamide der Formel (I) weisen mikrobizide Eigenschaften auf. Vor allem sind sie fungizid und bakterizid wirksam und einsetzbar vorzugsweise im Pflanzenschutz und im Materialschutz. Überraschenderweise zeigen die erfindungsgemässen Verbindungen eine bessere mikrobizide Wirksamkeit als vorbekannte und fungizid wirksame Verbindungen. Die erfindungsgemässen Stoffe stellen somit eine Bereicherung der Technik dar.

Die erfindungsgemässen N-Jodpropargylchlormethansulfonamide sind durch die Formel (I) allgemein definiert.

In ihr steht vorzugsweise

R für Alkyl mit 1 bis 6 Kohlenstoffatomen oder für gegebenenfalls ein- bis fünffach, gleiche oder verschieden durch Fluor, Chlor, Brom, Jod, Alkyl mit 1 bis 4 Kohlenstoffatomen und Halogenalkyl mit 1 bis 4 Kohlenstoffatomen und 1 bis 5 gleichen oder verschiedenen Halogenatomen substituiertes Phenyl.

Besonders bevorzugt sind die Verbindungen der Formel (I), in denen

R für Alkyl mit 1 bis 4 Kohlenstoffatomen, wie Methyl, Ethyl, n- und iso-Propyl, n-, sec-, iso- und tert.-Butyl, oder für gegebenenfalls ein- bis dreifach, gleich oder verschieden durch Fluor, Chlor, Alkyl mit 1 bis 3 Kohlenstoffatomen, wie Methyl, Ethyl, n- oder iso-Propyl, und Halogenalkyl mit 1 oder 2 Kohlenstoffatomen und 1 bis 5 gleichen oder verschiedenen Halogenatomen, wie Chlor und Fluor, substituiertes Phenyl steht.

Ganz besonders bevorzugt sind die Verbindungen der Formel (I), in denen

R für Methyl, Ethyl, iso-Propyl, tert.-Butyl, Phenyl, 3-Chlorphenyl, 4-Chlorphenyl, 2-Chlorphenyl, 4-Fluorphenyl, 3,4-Dichlorphenyl, 3,5-Dichlorphenyl, 2,3-Dichlorphenyl, 2,4,6-Trichlorphenyl, 2-Methylphenyl, 3-Methylphenyl, 4-Methylphenyl, 2,6-Dimethylphenyl, 2,3-Dimethylphenyl, 3-Trifluormethylphenyl, 2-Methyl-6-ethylphenyl, 4-Trifluormethylphenyl, 3,5-Di-(trifluormethyl)-phenyl und 4-Trichlormethylphenyl steht.

Der Reaktionsablauf der erfindungsgemässen Verfahrens zur Herstellung der neuen Verbindungen kann durch das folgende Formelschema wiedergegeben werden, wenn man als Ausgangsstoffe N-Propargyl-N-phenylchlormethansulfonamid und Jod in Gegenwart einer Base verwendet:

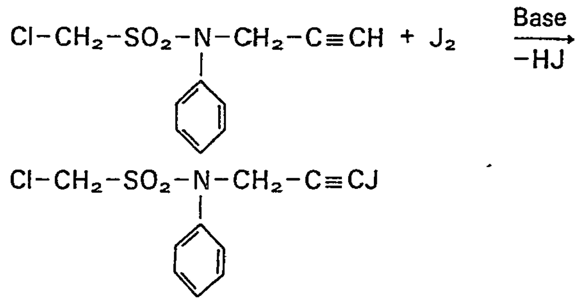


Die bei der Durchführung des erfindungsgemässen Verfahrens als Ausgangsstoffe zu verwendenden N-Propargyl-chlormethansulfonamide sind durch die Formel (II) allgemein definiert. Diese Verbindungen die Bedeutungen, die R hat auch in diesen Verbindungen die Bedeutungen, die bereits dafür bei der Formel (I) genannt wurden. Sie können aber nach Analogieverfahren hergestellt werden, z.B. in dem man

a) Amine der Formel (III)

$R-NH_2$ (III)

mit Propargylhalogeniden der Formel (IV)

$HC\equiv C-CH_2-X$ (IV)

gegebenenfalls in Gegenwart von Basen zu N-Propargylaminen der Formel (V)

$$R-NH-CH_2-C\equiv CH \qquad (V)$$

umsetzt und diese mit Chlormethansulfochlorid der Formel (VI)

$$Cl-CH_2-SO_2-Cl \qquad (VI)$$

gegebenenfalls in Gegenwart von Basen zu den als Ausgangsverbindungen einzusetzenden N-Propargylchlormethansulfonamiden der Formel (II) umsetzt, wobei in den obigen Formeln (II) bis (VI) R die angegebene Bedeutung hat und

X für Halogen, vorzugsweise für Chlor oder Brom, steht oder wenn man

b) die Amine der Formel (III)

$$R-NH_2 \qquad (III)$$

mit Chlormethansulfochlorid der Formel (VI)

$$Cl-CH_2-SO_2-Cl \qquad (VI)$$

gegebenenfalls in Gegenwart von Basen zu den Chlormethansulfonamiden der Formel (VII)

$$Cl-CH_2-SO_2-\underset{\displaystyle R}{\underset{|}{NH}} \qquad (VII)$$

umsetzt, diese mit Basen in die Salze (VIII)

$$Cl-CH_2-SO_2-\underset{\displaystyle R}{\underset{|}{\overline{N}^{\ominus}}}A^{\oplus} \qquad (VIII)$$

und anschliessend mit Propargylhalogeniden der Formel (IV) zu den gewünschten Ausgangsverbindungen der Formel (II) umsetzt, wobei in den obigen Formeln R die oben angegebene Bedeutung hat und $A^{\oplus}$ für ein Alkalimetalläquivalent steht.

Die Umsetzungen zu den Verbindungen der Formel (II) können in Lösungsmitteln durchgeführt werden, die unter den gegebenen Reaktionsbedingungen gegen die Reagentien und die verwendeten Basen inert sind.

So können die Umsetzungen z.B. von Verbindungen der Formel (III) und (V) mit (IV) bzw. (VI) z.B. in Toluol, Dichlorbenzol, Tetrahydrofuran, Dimethylformamid u.a. durchgeführt werden, als Basen kommen z.B. Amine, wie Triethylamin oder Pyridin, oder Alkalihydroxide bzw. -carbonate, wie Natriumhydroxid und Kaliumcarbonat, in Betracht.

Die Überführung der Verbindungen der Formel (VII) in die entsprechenden Salze der Formel (VII) erfolgt mit starken Basen, wie z.B. Natriumhydroxid, Natriummethylat und Kalium-tert.-butylat, und gegebenenfalls in Gegenwart inerter Lösungsmittel, wie z.B. Tetrahydrofuran und Dimethylformamid, in denen auch die anschliessende Alkylierungsreaktion, die Umsetzung mit Propargylhalogeniden, zu den Ausgangsverbindungen der Formel (II) durchgeführt werden kann.

Es kann z.B. bei der Umsetzung der Amine der Formel (III) mit dem Chlormethansulfochlorid der Formel (VI) zweckmässig sein ohne eins der aufgezählten Lösungsmittel und eine der aufgezählten Basen zu arbeiten und das Amin der Formel (III) selbst im Überschuss zu verwenden.

Die Alkylierungs- und Sulfonierungsreaktionen werden im allgemeinen bei Temperaturen von 0° C bis 120° C durchgeführt.

Für die erfindungsgemässe Umsetzung der Verbindungen der Formel (II) mit Jod werden als Lösungsmittel vorzugsweise Alkohole, wie Methanol und Butanol, eingesetzt. Als Basen werden bevorzugt Alkalihydroxide und Alkoholate, wie Natriumhydroxid und Natriummethylat, verwendet.

Die Reaktionstemperatur bei dem erfindungsgemässen Jodierungsverfahren liegt zwischen −20° C und +20° C.

Die Isolierung und Reinigung der Umsetzungsprodukte sowohl der Ausgangs- als auch der Endprodukte, erfolgt durch bekannte Methoden, wie z.B. Destillation, Kristallisation und Chromatographie.

Die zur Herstellung der Ausgangsverbindunden der Formel (II) benötigten Verbindungen der Formeln (III) bis (VIII) sind grösstenteils allgemein bekannte Verbindungen.

Die erfindungsgemässen Wirkstoffe weisen eine starke mikrobizide Wirkung auf und können zu Bekämpfung von unerwünschten Mikroorganismen praktisch eingesetzt werden. Die Wirkstoffe sind für den Gebrauch als Pflanzenschutzmittel und als mikrobizide Mittel zum Schutz technischer Materialien geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:

Botrytis-Arten, wie beispielsweise Botrytis cinerea;

Plasmopara-Arten, wie beispielsweise Plasmopara viticola;

Uromyces-Arten, wie beispielsweise Uromyces appendiculatus;

Sphaerotheca-Arten, wie beispielsweise Sphaerotheca fuliginea;

Venturia-Arten, wie beispielsweise Venturia inaequalis;

Podosphaera-Arten, wie beispielsweise Podosphaera leucotricha;

Phytophthora-Arten, wie beispielsweise Phytophthora infestans;

Erysiphe-Arten, wie beispielsweise Erysiphe graminis;

Puccinia-Arten, wie beispielsweise Puccinia recondita;

Fusarium-Arten, wie beispielsweise Fusarium culmorum;

Ustilago-Arten, wie beispielsweise Ustilago nuda oder avenae;

Septoria-Arten, wie beispielsweise Systoria nodorum;

Tilletia-Arten, wie beispielsweise Tilletia caries;

Pyricularia-Arten, wie beispielsweise Pyricularia oryzae;

Pellicularia-Arten, wie beispielsweise Pellicularia sasakii;

Pyrenophora-Arten, wie beispielsweise Pyrenophora teres (Konidienform Drechslera, Synonym Helmisthosporium);

Cochliobolus-Arten, wie beispielsweise Cochliobolus sativus (Konidienform Drechslera, Synonym Helminthosporium);

Leptosphaeria-Arten, wie beispielsweise Leptosphaeria nodorum;

Cercospora-Arten, wie beispielsweise Cerospora canescens.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemässen Wirkstoffe mit besonders gutem Erfolg zur Bekämpfung der Erreger von Plasmopara viticola, Puccinia recondita, Leptosphaeria nodorum und auch Pyricularia oryzae eingesetzt werden.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel. Als Emulgier- und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie Eiweisshydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemässen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und Wachstumsregulatoren.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Giessen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem grösseren Bereich variiert werden. Sie liegen im allgemeinen zwischen 1 und 0,0001 Gew.-%, vorzugsweise zwischen 0,5 und 0,001%.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02%, am Wirkungsort erforderlich.

Technische Materialien im Rahmen der vorliegenden Erfindung sind Produkte, die selbst nicht in der Natur vorkommen, sondern aus natürlichen oder synthetischen Ausgangsmaterialien gefertigt werden. Die zu schützenden Produkte im Rahmen der vorliegenden Erfindung sind technische Materialien, die von Mikroorganismen befallen und/ oder zersetzt werden können.

Technische Materialien, die durch die erfindungsgemässen Substanzen vor einer mikrobiellen Veränderung und Zerstörung geschützt werden sollen, sind beispielsweise Klebstoffe, Leime, Papiere und Kartone, Textilien, Leder, Holz, Anstrichmittel, Putze, Kühlschmiermittel, Dichtungsmassen und Kunststoffartikel, die von Mikroorganismen befallen oder zersetzt werden können. Im Rahmen der zu schützenden Materialien seien auch Teile von Produktionsanlagen, wie beispielsweise Kühlwasser- und Kühlschmiermittelkreisläufe, genannt, deren Funktionstüchtigkeit durch Mikroorganismen beeinträchtigt werden kann. Vorzugsweise können die erfindungsgemässen Wirkstoffe zum Schutz von Klebstoffen, Papier, Karton, Anstrichfilmen, Holz u.ä. verwendet werden.

Mikroorganismen, die einen Abbau oder eine Veränderung der technischen Materialien bewirken können, sind beispielsweise Bakterien, Pilze, Hefen, Algen u. Schleimeorganismen. Vorzugsweise haben die erfindungsgemässen Substanzen eine starke und breite Wirkung gegen Pilze; von der fungiziden Wirkung werden Schimmelpilze ebenso erfasst wie Holz-zerstörende und Holzverfärbende Pilze.

Es seien beispielsweise Mikroorganismen der folgenden Gattungen genannt:

Alternaria, wie Alternaria tenuis,
Aspergillus, wie Aspergillus niger,
Aureobasidium, wie Aureobasidium pullulans,
Chaetomium, wie Chaetomium globosum,
Coniophora, wie Coniophora puteana,
Lentinus, wie Lentinus tigrinus,
Penicillium, wie Penicillium glaucum,
Polyporus, wie Polyporus versicolor,
Sclerophoma, wie Sclerophoma pityophila,
Staphylococcus, wie Staphylococcus aureus.

Je nach ihrem Anwendungsgebiet können die erfindungsgemässen Substanzen in die üblichen Formulierungen überführt werden, wie Lösungen, Emulsionen, Suspensionen, Pulver, Pasten und Granulate. Diese können in an sich bekannter Weise hergestellt werden, z.B. durch Vermischen der Wirkstoffe mit einem Streckmittel, also flüssigem Lösungsmittel und/oder festen Trägerstoffen, gegebenenfalls unter Verwendung von oberflächenaktiven Mitteln, wie Emulgatoren und/oder Dispergiermitteln, wobei beispielsweise im Falle der Benutzung von Verstreckmitteln, gegebenenfalls organische Solventien, als Hilfslösungsmittel verwendet werden können.

Organische Solventien für die Wirkstoffe können beispielsweise Alkohole, wie aliphatische niedere Alkohole, vorzugsweise Ethanol oder Isopropanol, oder Benzylalkohol, Ketone wir Aceton oder Methylethylketon, flüssige Kohlenwasserstoffe, wie Benzinfraktionen, halogenierte Kohlenwasserstoffe, wie 1,2-Dichlorethan sein.

Die Anwendungskonzentration der erfindungsgemässen Substanzen richtet sich nach der Art und dem Vorkommen der zu bekämpfenden Mikroorganismen, sowie nach der Zusammensetzung des zu schützenden Materials. Die optimale Einsatzmenge kann durch Testreihen ermittelt werden. Im allgemeinen liegen die Anwendungskonzentrationen im Bereich von 0,001 bis 5 Gew.-%, vorzugsweise von 0,1 bis 1 Gew.-%, bezogen auf das zu schützende Material.

Die erfindungsgemässen neuen Wirkstoffe können auch in Mischung mit anderen bekannten Wirkstoffen vorliegen. Beispielsweise seien die folgenden Wirkstoffe genannt:

Benzylalkoholmono(poly)hemiformal, Benzimidazolyl-methylcarbamate, Tetramethylthiuramdisulfid, Zinksalze von Dialkylthiocarbamaten, 2,4,5,6-Tetrachlorisophthalonitril, Thiazolylbenzimidazol, Mercaptobenzthiazol, Trialkylzinnverbindungen, Methylenbisthiocyanate und Phenolderivate, wie 2-Phenylphenol, (2,2'-Dihydroxy-5,5'-dichlor)-diphenylmethan, 3-Methyl-4-chlor-phenol, ferner quaternäre Ammoniumsalze, Isothiazolinone, Benzisothiazolinon.

Das erfindungsgemässe Herstellungsverfahren sei an den folgenden Beispielen aufgezeigt:

($^1H$-NMR-Daten: Lösungsmittel $CDCl_3$, TMS als innerer Standard)

*Beispiel 1*

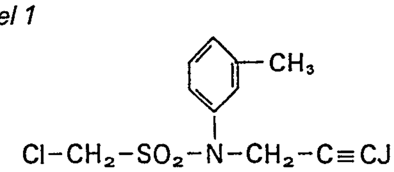

Zu 1070 g 3-Aminotoluol werden bei 90° C 372 g 3-Chlor-1-propin zugetropft. Man rührt 7 h, versetzt mit Wasser, schüttelt mit Dichlormethan aus und engt ein. Das resultierende Öl wird fraktioniert destilliert, wobei 390 g N-Propargyl-3-methyl-anilin als Öl mit dem Siedepunkt 90-105° C/1,5 mbar erhalten werden.

580 g N-Propargyl-3-methyl-anilin werden in 2 l Tetrahydrofuran gelöst, 740 g Triethylamin zugesetzt und bei 20-70° C insgesamt 1750 g 90% Chlormethansulfochlorid binnen 40 h zugesetzt. Man versetzt mit Wasser, trennt die organische Phase ab, wäscht mit verdünnter Salzsäure und Wasser und engt ein. Nach Filtration über Kieselgel kristallisieren aus dem Öl 515 g N-Propargyl-N-(3-methylphenyl)-chlormethansulfonamid mit dem Schmelzpunkt 48° C.

515 g N-Propargyl-N-(3-methylphenyl)-chlormethansulfonamid wurden in 5 l Methanol gelöst und bei 0-5° C eine Lösung von 120 g Natriumhydroxid in 120 g Wasser zugetropft. Danach werden 580 g Iod zugesetzt. Man rührt noch 4 h, fällt mit Wasser, saugt ab und kristallisiert aus Ethanol um. Ausbeute 486 g N-Iodpropargyl-N-(3-methylphenyl)-chlormethansulfonamid mit den Schmelzpunkt 92-94° C.

$^1$H-NMR: δ = 2,38 (s, 3H); 4,53 (s, 2H); 4,64 (s, 2H); 7,1-7,4 (m, 4H).

*Beispiel 2*

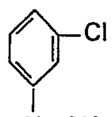

$Cl-CH_2-SO_2-N-CH_2-C{\equiv}CJ$

Zu 637,5 g 3-Chloranilin werden bei Raumtemperatur 815 g (90%ig) Chlormethansulfochlorid getropft. Man rührt noch 12 h, saugt die Kristalle ab und wäscht mit Wasser, wobei 320 g N-(3-Chlorphenyl)-chlormethansulfonamid zurückblieben mit einem Schmelzpunkt von 98° C.

Diese werden in 500 ml Tetrahydrofuran gelöst und eine Lösung von 183,6 g Kalium-tert.-butylat in 1 l Tetrahydrofuran bei Raumtemperatur zugetropft. Der nach Abziehen des Tetrahydrofurans verbleibende Rückstand wird in 1 l Dimethylformamid gelöst und eine Lösung von 128 g 3-Chlor-1-propin in 200 ml Dimethylformamid zugetropft. Man rührt noch 8 h bei 60° C, engt ein, nimmt in Methylenchlorid auf, wäscht mit Wasser und engt ein. Der Rückstand wird in 2 l Methanol gelöst. Bei 0-5° C werden 250 g 25% Natriumhydroxid und 210 g Iod zugesetzt und noch 3 h gerührt. Der Niederschlag wird abgesaugt und aus Ethanol umkristallisiert. Ausbeute 204 g N-Iodpropargyl-N-(3-chlorphenyl)-chlormethansulfonamid mit einem Schmelzpunkt von 111-114° C.

$^1$H-NMR: δ = 4,57 (s, 2H); 4,64 (s, 2H); 7,2-7,6 (m, 4H).

Ebenso wurden die Verbindungen der Formel (I)

$$Cl-CH_2-SO_2-\underset{\displaystyle R}{\underset{|}{N}}-CH_2-C{\equiv}CI \qquad (I)$$

hergestellt:

| Bsp. Nr. | R | Schmelzpunkt [°C] | $^1$H-NMR-Daten: δ= |
|---|---|---|---|
| 3 | $-CH_3$ | 47 | 3,12 (s, 3H); 4,30 (s, 2H); 4,61 (s, 2H) |
| 4 | 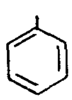 | 70 | 4,55 (s, 2H); 4,67 (s, 2H); 7,3-7,6 (m, 5H) |
| 5 | 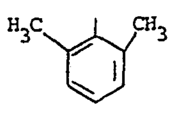 | 101 | 2,41 (s, 6H); 4,58 (s, 2H); 4,60 (s, 2H); 7,1 (m, 3H) |
| 6 | 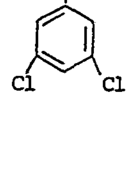 | 126 | 4,58 (s, 2H); 4,64 (s, 2H); 7,3-7,5 (m, 3H) |
| 7 | 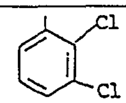 | 106 | 4,12 (s, 4H); 7,0-7,3 (m, 3H) |
| 8 | 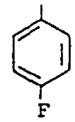 | 46 | 4,56 (s, 2H); 4,65 (s, 2H); 6,9-7,7 (m, 4H) |
| 9 | 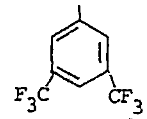 | — | 4,65 (s, 2H); 4,70 (s, 2H); 7,8-8,1 (m, 3H) |

In den nachfolgenden Beispielen wird die nachfolgende Verbindung, die ein bekanntes Fungizid ist, als Vergleichssubstanz eingesetzt:

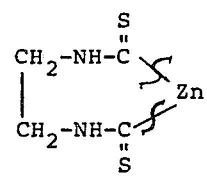

Zink-ethylen-1,2-bis-(dithiocarbamat)

*Beispiel A*

*Puccinia-Test (Weizen)/protektiv*

Lösungsmittel: 100 Gewichtsteile Dimethylformamid

Emulgator: 0,25 Gewichtsteile Alkylarylpolyglykolether

Zur Herstellung einer zweckmässigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit einer Sporensuspension von Puccinia recondita in einer 0,1%igen wässerigen Agarlösung inokuliert. Nach Antrocknen besprüht man die Pflanzen mit der Wirkstoffzubereitung taufeucht. Die Pflanzen verbleiben 24 Stunden bei 20° C und 100% rel. Luftfeuchtigkeit in einer Inkubationskabine.

Die Pflanzen werden in einem Gewächshaus bei einer Temperatur von ca. 20° C und einer relativen Luftfeuchtigkeit von ca. 80% aufgestellt, um die Entwicklung von Rostpusteln zu begünstigen.

10 Tage nach der Inokulation erfolgt die Auswertung.

Eine deutliche Überlegenheit in der Wirksamkeit gegenüber dem Stand der Technik zeigt bei diesem Test z.B. die Verbindung gemäss dem Herstellungsbeispiel 1.

*Beispiel B*

*Plasmopara-Test (Reben)/protektiv*

Lösungsmittel: 4,7 Gewichtsteile Aceton

Emulgator: 0,3 Gewichtsteile Alkylarylpolyglykolether

Zur Herstellung einer zweckmässigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wässerigen Sporensuspension von Plasmopara viticola inokuliert und verbleiben dann 1 Tag in einer Feuchtkammer bei 20 bis 22° C und 100% relativer Luftfeuchtigkeit. Anschliessend werden die Pflanzen 5 Tage im Gewächshaus bei 22° C und ca. 80% Luftfeuchtigkeit aufgestellt. Die Pflanzen werden dann angefeuchtet und 1 Tag in eine Feuchtkammer gestellt.

7 Tage nach der Inokulation erfolgt die Auswertung.

Eine deutliche Überlegenheit in der Wirksamkeit gegenüber dem Stand der Technik zeigen in diesem Test z.B. die Verbindungen gemäss den Herstellungsbeispielen: 1 und 8.

*Beispiel C*

*Leptosphaeria nodorum-Test (Weizen)/protektiv*

Lösungsmittel: 100 Gewichtsteile Dimethylformamid

Emulgator: 0,25 Gewichtsteile Alkylarylpolyglykolether

Zur Herstellung einer zweckmässigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Leptosphaeria nodorum besprüht. Die Pflanzen verbleiben 48 Stunden bei 20° C und 100% relativer Luftfeuchtigkeit in einer Inkubationskabine.

Die Pflanzen werden in einem Gewächshaus bei einer Temperatur von ca. 15° C und einer relativen Luftfeuchtigkeit von ca. 80% aufgestellt.

10 Tage nach der Inokulation erfolgt die Auswertung.

Eine deutliche Überlegenheit in der Wirksamkeit gegenüber dem Stand der Technik zeigt bei diesem Test z.B. die Verbindung gemäss dem Herstellungsbeispiel 1.

*Beispiel D*

Zum Nachweis der Wirksamkeit gegen Pilze werden die minimalen Hemm-Konzentrationen (MHK) von erfindungsgemässen Wirkstoffen bestimmt:

Ein Agar, der aus Bierwürze und Pepton hergestellt wird, wird mit erfindungsgemässen Wirkstoffen in Konzentrationen von 0,1 mg/l bis 5.000 mg/l versetzt. Nach Erstarren des Agars erfolgt Kontamination mit Reinkulturen der in der Tabelle aufgeführten Testorganismen.

Nach 2-wöchiger Lagerung bei 28° C und 60 bis 70% rel. Luftfeuchtigkeit wird die MHK bestimmt. MHK ist die niedrigste Konzentration an Wirkstoff, bei der keinerlei Bewuchs durch die verwendete Mikrobenart erfolgt. Gute Wirkungen zeigten die Verbindungen gemäss Herstellungsbeispielen 1 und 2.

Tabelle:
Alternaria tenuis
Aspergillus niger
Aureobasidium pullulans
Chaetomium globosum
Coniophora puteana
Lentinus tigrinus
Penicillium glaucum
Polyporus versicolor
Sclerophoma pityophila
Trichoderma viride

*Beispiel E*

Die erfindungsgemässe Substanz gemäss Beispiel 2 wird in Konzentrationen von jeweils 0,1 bis 100 mg/l in Allens Nährlösung (Arch. Mikrobiol, *17,* 35 bis 53 (1952)), die in 4 l sterilem Wasser, 0,2 g Ammoniumchlorid, 4,0 g Calciumchlorid, 2,05 g Magnesiumsulfat, 0,02 g Eisenchlorid und 1% Caprolactam enthält, in wenig Aceton gelöst, zur Anwendung gebracht. Kurz vorher wird die Nährlösung mit Schleimorganismen (ca. $10^6$ Keime/ml), die aus bei der Polyamid-Herstellung verwendeten Spinnwasserkreisläufen isoliert wurden, infiziert. Nährlösungen, die die minimale Hemmkonzentration (MHK) oder grössere Wirkstoffkonzentrationen aufweisen, sind nach 3-wöchiger Kultur bei Raumtemperatur noch völlig klar, d.h. die in wirkstofffreien Nährlösungen nach 3 bis 4 Tagen bemerkbare starke Vermehrung der Mikroben und Schleimbildung unterbleibt.

Für den Wirkstoff gemäss Beispiel 2 wird auf diese Weise eine MHK von 30 mg/l ermittelt.

**Patentansprüche**

1. N-Jodpropargyl-chlormethansulfonamide der Formel (I)

$$Cl-CH_2-SO_2-\underset{\substack{|\\ R}}{N}-CH_2-C\equiv CJ \qquad (I)$$

in welcher

R für Alkyl oder gegebenenfalls ein- bis mehrfach, gleich oder verschieden durch Alkyl, Halogen und Halogenalkyl substituiertes Aryl steht.

2. N-Jodpropargyl-chlormethansulfonamide gemäss Anspruch 1, wobei in der Formel (I)

R für Alkyl mit 1 bis 6 Kohlenstoffatomen oder für gegebenenfalls ein- bis fünffach, gleich oder verschieden durch Fluor, Chlor, Brom, Jod, Alkyl mit 1 bis 4 Kohlenstoffatomen und Halogenalkyl mit 1 bis 4 Kohlenstoffatomen und 1 bis 5 gleichen oder verschiedenen Halogenatomen substituiertes Phenyl steht.

3. N-Jodpropargyl-chlormethansulfonamide gemäss Anspruch 1, wobei in der Formel (I)

R für Alkyl mit 1 bis 4 Kohlenstoffatomen oder für gegebenenfalls ein- bis dreifach, gleich oder

verschieden durch Fluor, Chlor, Alkyl mit 1 bis 3 Kohlenstoffatomen und Halogenalkyl mit 1 oder 2 Kohlenstoffatomen und 1 bis 5 gleichen oder verschiedenen Halogenatomen substituiertes Phenyl steht.

4. N-Jodpropargyl-chlormethansulfonamide gemäss Anspruch 1, wobei in der Formel (I)

R für Methyl, Ethyl, iso-Propyl, tert.-Butyl, Phenyl, 2-, 3- oder 4-Chlorphenyl, 4-Fluorphenyl, 3,4-, 3,5- oder 2,3-Dichlorphenyl, 2,4,6-Trichlorphenyl, 2-, 3- oder 4-Methylphenyl, 2,3- oder 2,6-Dimethylphenyl, 2-Methyl-6-ethylphenyl, 3- oder 4-Trifluormethylphenyl, 3,5-Di-(trifluormethyl)-phenyl oder 4-Trichlormethylphenyl steht.

5. Verfahren zur Herstellung von N-Jodpropargyl-chlormethansulfonamiden der Formel (I)

$$\mathrm{Cl-CH_2-SO_2-\underset{\substack{|\\ R}}{N}-CH_2-C\equiv CJ} \qquad (I)$$

in welcher

R für Alkyl oder gegebenenfalls ein- bis mehrfach, gleich oder verschieden durch Alkyl, Halogen und Halogenalkyl substituiertes Aryl steht, dadurch gekennzeichnet, dass man N-Propargylchlormethansulfonamide der Formel (II)

$$\mathrm{Cl-CH_2-SO_2-\underset{\substack{|\\ R}}{N}-CH_2-C\equiv CH} \qquad (II)$$

in welcher

R die oben angegebene Bedeutung hat, in Gegenwart einer Base und gegebenenfalls in Gegenwart eines Lösungs- oder Verdünnungsmittels mit Jod umsetzt.

6. Mikrobizide Mittel, gekennzeichnet durch einen Gehalt an mindestens einem N-Jodpropargylchlormethansulfonamid der Formel (I).

7. Verwendung von N-Jodpropargylchlormethansulfonamiden der Formel (I) zur Bekämpfung von Mikroorganismen.

8. Verfahren zur Bekämpfung von Mikroorganismen, dadurch gekennzeichnet, dass man N-Jodpropargyl-chlormethansulfonamide der Formel (I) auf Mikroorganismen und/oder ihren Lebensraum einwirken lässt.

9. Verfahren zur Herstellung von mikrobiziden Mitteln, dadurch gekennzeichnet, dass man N-Jodpropargyl-chlormethansulfonamide der Formel (I) mit Streckmitteln und/oder oberflächenaktiven Mitteln vermischt.

10. N-Propargyl-chlormethansulfonamide der Formel (II)

$$\mathrm{Cl-CH_2-SO_2-\underset{\substack{|\\ R}}{N}-CH_2-C\equiv CH} \qquad (II)$$

in welcher

R für Alkyl oder für gegebenenfalls ein- bis mehrfach, gleich oder verschieden durch Alkyl, Halogen und Halogenalkyl substituiertes Aryl steht.

**Revendications**

1. N-iodopropargyl-chlorométhane-sulfonamides de formule (I)

$$\mathrm{Cl-CH_2-SO_2-\underset{\substack{|\\ R}}{N}-CH_2-C\equiv CI} \qquad (I)$$

dans laquelle

R représente un groupe alkyle ou un groupe aryle portant éventuellement un ou plusieurs substituants identiques ou différents choisis parmi les groupes alkyles, les halogènes et les groupes halogénoalkyles.

2. N-iodopropargyl-chlorométhane-sulfonamides selon la revendication 1, répondant à la formule (I), dans laquelle R représente un groupe alkyle en $C_1$-$C_6$ ou phényle portant éventuellement un à cinq substituants identiques ou différents choisis parmi le fluor, le chlore, le brome, l'iode, les groupes alkyles en $C_1$-$C_4$ et les groupes halogénoalkyles contenant 1 à 4 atomes de carbone et 1 à 5 atomes d'halogènes identiques ou différents.

3. N-iodopropargyl-chlorométhane-sulfonamides selon la revendication 1, répondant à la formule (I), dans laquelle R représente un groupe alkyle en $C_1$-$C_4$ ou phényle portant éventuellement un à trois substituants identiques ou différents choisis parmi le fluor, le chlore, les groupes alkyles en $C_1$-$C_3$ et halogénoalkyles contenant 1 ou 2 atomes de carbone et 1 à 5 atomes d'halogènes identiques ou différents.

4. N-iodopropargyl-chlorométhane-sulfonamides selon la revendication 1, répondant à la formule (I), dans laquelle R représente un groupe méthyle, éthyle, isopropyle, tert.-butyle, phényle, 2-, 3- ou 4-chlorophényle, 4-fluorophényle, 3,4-, 3,5- ou 2,3-dichlorophényle, 2,4,6-trichlorophényle, 2-, 3- ou 4-méthylphényle, 2,3- ou 2,6-diméthylphényle, 2-méthyl-6-éthylphényle, 3- ou 4-trifluorométhylphényle, 3,5-di-(trifluorométhyl)-phényle ou 4-trichlorométhylphényle.

5. Procédé de préparation des N-iodopropargyl-chlorométhane-sulfonamides de formule (I)

$$\mathrm{Cl-CH_2-SO_2-\underset{\substack{|\\ R}}{N}-CH_2-C\equiv CI} \qquad (I)$$

dans laquelle

R représente un groupe alkyle ou un groupe aryle portant éventuellement un ou plusieurs substituants identiques ou différents choisis parmi les groupes alkyles, les halogènes et les groupes halogénoalkyles, caractérisé en ce que l'on fait réagir des N-propargyl-chlorométhane-sulfonamides de formule (II)

$$\mathrm{Cl-CH_2-SO_2-\underset{\substack{|\\ R}}{N}-CH_2-C\equiv CH} \qquad (II)$$

dans laquelle

R a les significations indiquées ci-dessus, en

présence d'une base et éventuellement en présence d'un solvant ou diluant, avec l'iode.

6. Produit microbicide, caractérisé en ce qu'il contient au moins un N-iodopropargyl-chlorométhane-sulfonamide de formule (I).

7. Utilisation des N-iodopropargyl-chlorométhane-sulfonamides de formule (I) dans la lutte contre les micro-organismes.

8. Procédé pour lutter contre les micro-organismes, caractérisé en ce que l'on fait agir sur les micro-organismes et/ou leur habitat des N-iodopropargyl-chlorométhane-sulfonamides de formule (I).

9. Procédé de préparation de produits microbicides, caractérisé en ce que l'on mélange des N-iodopropargyl-chlorométhane-sulfonamides de formule (I) avec des diluants et/ou des agents tensioactifs.

10. N-propargyl-chlorométhane-sulfonamide de formule (II)

$$Cl-CH_2-SO_2-\underset{\displaystyle R}{\underset{|}{N}}-CH_2-C\equiv CH \quad (II)$$

dans laquelle

R représente un groupe alkyle ou un groupe aryle portant éventuellement un ou plusieurs substituants identiques ou différents choisis parmi les groupes alkyles, les halogènes et les groupes halogénoalkyles.

## Claims

1. N-Iodopropargyl-chloromethanesulphonamides of the formula (I)

$$Cl-CH_2-SO_2-\underset{\displaystyle R}{\underset{|}{N}}-CH_2-C\equiv CJ \quad (I)$$

in which

R represents alkyl, or aryl which is optionally monosubstituted to polysubstituted by identical or different substituents from the group comprising alkyl, halogen and halogenoalkyl.

2. N-Iodopropargyl-chloromethanesulphonamides according to Claim 1, wherein, in formula (I),

R represents alkyl with 1 to 6 carbon atoms, or phenyl which is optionally mono- to pentasubstituted by identical or different substituents from the group comprising fluorine, chlorine, bromine, iodine, alkyl with 1 to 4 carbon atoms and halogenoalkyl with 1 to 4 carbon atoms and 1 to 5 identical or different halogen atoms.

3. N-Iodopropargyl-chloromethanesulphonamides according to Claim 1, wherein, in formula (I),

R represents alkyl with 1 to 4 carbon atoms or phenyl which is optionally mono- to trisubstituted by identical or different substituents from the group comprising fluorine, chlorine, alkyl with 1 to 3 carbon atoms and halogenoalkyl with 1 or 2 carbon atoms and 1 to 5 identical or different halogen atoms.

4. N-Iodopropargyl-chloromethanesulphonamides according to Claim 1, wherein, in formula (I),

R represents methyl, ethyl, iso-propyl, tert.-butyl, phenyl, 2-, 3- or 4-chlorophenyl, 4-fluorophenyl, 3,4-, 3,5- or 2,3-dichlorophenyl, 2,4,6-trichlorophenyl, 2-, 3- or 4-methylphenyl, 2,3- or 2,6-dimethylphenyl, 2-methyl-6-ethylphenyl, 3- or 4-trifluoromethylphenyl, 3,5-di-(trifluoromethyl)-phenyl or 4-trichloromethyl-phenyl.

5. Process for the preparation of N-iodo-propargyl-chloromethanesulphonamides of the formula (I)

$$Cl-CH_2-SO_2-\underset{\displaystyle R}{\underset{|}{N}}-CH_2-C\equiv CJ \quad (I)$$

in which

R represents alkyl or aryl which is optionally monosubstituted to polysubstituted by identical or different substituents from the group comprising alkyl, halogen and halogenoalkyl, characterised in that N-propargyl-chloromethanesulphonamides of the formula (II)

$$Cl-CH_2-SO_2-\underset{\displaystyle R}{\underset{|}{N}}-CH_2-C\equiv CH \quad (II)$$

in which

R has the abovementioned meaning, are reacted with iodine in the presence of a base and, if appropriate, in the presence of a solvent or diluent.

6. Microbicidal agents, characterised in that they contain at least one N-iodopropargyl-chloromethanesulphonamide of the formula (I).

7. Use of N-iodopropargylchloromethanesulphonamides of the formula (I) for combating microorganisms.

8. Method of combating microorganisms, characterised in that N-iodopropargyl-chloromethanesulphonamides of the formula (I) are allowed to act on microorganisms and/or their environment.

9. Process for the preparation of microbicidal agents, characterised in that N-iodopropargyl-chloromethanesulphonamides of the formula (I) are mixed with extenders and/or surface-active agents.

10. N-propargyl-chloromethanesulphonamides of the formula (II)

$$Cl-CH_2-SO_2-\underset{\displaystyle R}{\underset{|}{N}}-CH_2-C\equiv CH \quad (II)$$

in which

R represents alkyl or aryl which is optionally monosubstituted to polysubstituted by identical or different substituents from the group comprising alkyl, halogen and halogenoalkyl.